(19) Europäisches Patentamt
European Patent Office
Office européen des brevets

(11) Publication number: **0 280 232 B1**

(12) # EUROPEAN PATENT SPECIFICATION

(45) Date of publication of patent specification: **10.02.93**

(51) Int. Cl.[5]: **C12P 7/62**, C12P 17/04, //(C12P7/62,C12R1:15), (C12P17/04,C12R1:15)

(21) Application number: **88102567.0**

(22) Date of filing: **22.02.88**

(54) **Monoacetylation of diols using a biocatalyst from corynebacterium oxydans.**

(30) Priority: **24.02.87 US 18254**

(43) Date of publication of application:
**31.08.88 Bulletin 88/35**

(45) Publication of the grant of the patent:
**10.02.93 Bulletin 93/06**

(84) Designated Contracting States:
**AT CH DE FR GB IT LI SE**

(56) References cited:
**DE-A- 1 947 038**
**FR-A- 2 072 136**

**CHEMICAL ABSTRACTS, vol. 95, no. 4, August 1981, page 534, abstract no. 59937a, Columbus, Ohio, US; && JP-A-81 21 593 (NITTO CHEMICAL INDUSTRY CO. LTD) 28-02-1981**

(73) Proprietor: **GENENCOR INTERNATIONAL, INC.**
**180 Kimball Way**
**South San Francisco, CA 94080(US)**

(72) Inventor: **Green, Frederick Richard III Eastman Kodak Company**
**Patent Department 343 State Street**
**Rochester New York 14650(US)**
Inventor: **Olyslager, Robert James Eastman Kodak Company**
**Patent Department 343 State Street**
**Rochester New York 14650(US)**

(74) Representative: **Brandes, Jürgen, Dr. et al**
**Wuesthoff & Wuesthoff Patent- und Rechtsanwälte Schweigerstrasse 2**
**W-8000 München 90(DE)**

**EP 0 280 232 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

In the preparation of many compounds, it is desirable to introduce a chiral center into the compound. Such sterospecific compounds are important for example, because only one stereo isomer of many compounds is biologically active. Methods for the introduction of a chiral center into a compound which can be used directly or as the precursor to another compound are therefore commercially important. The preparation of the monoacetate of an $\alpha$, $\omega$-diol would be an example of such a method.

Using non-enzymatic methods, it is very difficult to prepare the monoacetates from diol compounds, particularly where the hydroxy groups are similar. The diacetate is usually formed. Some methods are known, however, and reference is made to Pochini, Salerno and Ungaro, A New Simple Catalyst for the Synthesis of 1,3-Diols and Their Monoesters from Linear Aliphatic Aldehydes, Synthesis Vol 164 pg 164, 1975. No chiral compounds are produced according to this reference.

In the synthesis of microbial growth factor (also known as "(-)-A-Factor)" or biotin, one of the steps in the method involves the introduction of a chiral center. Such a method is described in Wang and Sih, Bifunctional Chiral Synthons via Biochemical Methods, 4. Chiral Precursors to (+)-Biotin and (-)-A-Factor, Tetrahedron Letters, Vol. 25, No. 4, pgs 4999-5002 (1984). According to the method of this reference, a prochiral diacetate is used. (The term "prochiral" refers to a compound that is potentially a chiral compound in that it will become a chiral compound by changing one group). An optically active monoacetate intermediate is obtained by the enantioselective hydrolysis of the prochiral diacetate. The enzyme pig pancreatic lipase or PPL was used for this purpose.

In another synthesis, this reference also describes starting with a diacetate compound. The optically active intermediate for biotin was prepared by the enzymatic conversion of a prochiral meso-diacetoxy ester. The enzyme used pig liver esterase or PLE.

Another reference describes a similar process. In US Patent 4,415,657, there is disclosed a method for the synthesis of an optically active monoalkyl ester of beta-(S)-aminoglutaric acid from the protected dialkyl ester of the acid. Culture broth, cells or treated cells of certain microorganisms are used. Corynebacterium sepedonicum and Corynebacterium xerosis are mentioned as possible sources of the necessary catalytic activity.

In US Patent 3,558,431 there is described a method for the oxidation of organic compounds using a Corynebacterium which is the preferred bacterium used in the present invention. In the method of this patent, pentarythritol is converted to tris(hydroxymethyl)acetic acid by fermentation with the organism. No monoacetates from diols are produced nor are any chiral compounds produced. This same microorganism is used in a similar process described in U. S. Patent 3,642,581. (The microorganism in these references is referred to as a Flavobacterium. It is now believed to be a Corynebacterium.)

Still further improvements in the production of monoacetates from diols and particularly chiral compounds are desired. In the case of the intermediate for microbial growth factor in particular, further improvements in the yield are needed. The yield in the above noted reference for the chiral intermediate from the diacetate compound was only 34%. The yield needs to be improved while maintaining the excellent enantiomeric excess (e.e.) achieved by the reference. (95:5)

According to the present invention there is provided a method for making a monoacetate from a diol, said method comprising the step of reacting said diol with an ester of acetic acid in the presence of a biocatalyst derived from Corynebacterium oxydans.

The method of the invention is particularly useful in the preparation of chiral compounds from prochiral diols.

The compounds which are produced in the method of the present invention are useful as intermediates in the synthesis of other compounds. For example, the chiral monoacetate in the synthetic pathway to (-)-A-Factor can be made with this method in high yield. This particular compound, as an example, can be made in 92% yield with an e.e. of 96:4. This is illustrated in the Examples which follow. The monoacetate can be further converted using conventional reactions into other optically active intermediates. For example the remaining hydroxyl group can be converted to halo such as chloro, bromo or iodo; azido; phthalimido; cyano; and carboxyl.

The method of the present invention is useful for the monoetylation of a wide variety of prochiral diols. Usefuls diols can be represented by the formula:

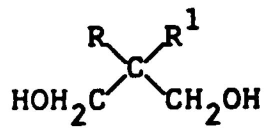

wherein

R and R′ are different and are selected from the group consisting of hydrogen; substituted or unsubstituted alkyl, such as methyl, propyl, chlorobutyl, isopropyl and t-butyl, alkoxy, substituted or unsubstituted benzyl, substituted or unsubstituted aryl, such as phenyl, chlorophenyl, naphthyl and substituted or unsubstituted heterocyclic, such as pyridyl as chloropyridyl.

Other useful diols which are not prochiral compounds but which can be monoetylated according to the present invention include: 1,4-butanediol and 1,5-pentanediol.

The biocatalyst that is used in the present method is derived from Corynebacterium oxydans. By "derived from", we mean that any composition that is made from this species of microorganism that catalyzes the monoacetylation reaction can be used. Useful compositions include the culture medium containing the cells, the recovered cells themselves or extracts from the cells which include the necessary catalytic activity. The method need not be carried out in the presence of viable cells as in a fermentation. The composition is used as the catalyst in the reaction.

The isolation, maintenance and characterization of a typical Corynebacterium oxydans is described in previously mentioned U. S. Patent 3,558,431. This particular strain, ATCC No. 21,245, is the currently preferred source of the biocatalyst in the present method. However, other strains of this species can also be used as is illustrated in the examples.

As noted, the present method is carried out with a mixture of a diol, an ester of acetic acid and a biocatalyst. It will be noted that no (or very little) water need be present. This essentially all organic reaction medium is unusual for reactions of this type. The water that is typically associated with the biocatalyst is sufficient although a reaction medium wherein the ester of the acetic acid is saturated with water is preferred. This requires only a small amount of water.

Any ester of acetic acid can be used. Typical examples are ethyl, methyl and butyl acetate. Ethyl acetate is preferred since higher yields are produced. The ester serves as both the solvent for the mixture and the source of the acetyl group in the acetylation. It is surprising that these compounds can serve as the source of the acetyl group since similar compounds cannot. In a similar reaction mixture, compounds such as dimethyl carbonate, methyl propionate and ethyl formate were not useful.

The reaction mixture can optionally contain small amounts of other materials. For example, the reaction mixture can contain sodium bicarbonate that removes the small amount of acid that might be formed from the reaction of water with the ester of acetic acid. A small amount of acid, preferably hydrochloric or phosphoric acid, or base, preferably sodium carbonate or sodium hydroxide can also be added to adjust the pH.

The ratio of the reactants in the reaction mixture can vary widely. The weight ratio of the ester of acetic acid and the diol can be between about 5:1 and 100:1. Preferably, the ester is present in excess and the ester to diol ratio is between 30:1 and 20:1. The amount of biocatalyst is also widely variable. Generally it is present in an amount of 0.1-10%, preferably 1%. Preferably, water can be present up to its saturation point in the ester.

The reaction conditions are not critical. The temperature is preferably between about 20° and 50°C. The pH is preferably between about 5 and 9. The reaction time can vary quite widely and due to the mild nature of the reaction conditions can be quite long. Reaction times between about 18h and 72h are typical.

In the examples which follow, the yield is calculated based on the moles of starting diol. The enantiomeric excess or "e.e." is determined from the $H^1$-n.m.r. spectra of the Mosher's esters (J. Org. Chem. 34, 2543:1969) of the free hydroxyl groups.

Recovery of the desired compound from the organic reaction medium is by conventional methods. Where the compound is to be used as an intermediate for another compound, recovery may not be necessary.

Preparation of Cornybacterium oxydans

Cornybacterium oxydans (ATCC 21245) was grown as follows:

1700 mL of a culture medium was prepared from glucose (17 g), yeast extract (17 g), potassium

hydrogen phosphate (3.4 g), and 17 mL of a salt solution prepared from a 1 L solution of $MgSO_4.7H_2O$ (0.25 g), $MnSO_4.7H_2O$ (0.17 g), $FeSO_4.\ 7H_2O$ (0.028 g), NaCl (0.0006 g), $CaCl_2.2H_2O$ (0.001 g), and $ZnSO_4.7H_2O$ (0.006 g). The pH was adjusted to 7 with potassium hydroxide.

The pure culture was used to innoculate two 25 mL samples of the above sterilized culture medium, which were then shaken at 30°C at 250 RPM for about 15 hours.

The flasks were combined and used to innoculate 500 mL of the above sterilized culture medium, which was then shaken at 30°C at 125 RPM for 24 hours.

The solution was then centrifuged at 9000 RPM for 20 minutes, and the resulting cell pellet was frozen at -20°C or lyophilized.

## Monoacetylation of Diols

A general procedure was used to convert the various diols into their corresponding monoacetylated derivatives: the appropriate diol (0.5 g) was dissolved in 25 mL of ethyl acetate containing 2.0 mL of water, in which about 0.2 g dry cells had been suspended.

The resulting reaction mixture was shaken at 300 RPM at 30°C until the reaction was essentially complete as determined by gas chromatographic analysis.

The cells were removed by filtration, and the filtrate was washed with saturated sodium bicarbonate solution. The solvent was dried and concentrated to yield the product, which could be purified by chromatography. The monoacetate structures were verified by nuclear magnetic resonance and gas chromatographic analysis.

The tables list the various diols, the yields of monoacetylated product, and, in some cases, their optical purities.

## Examples 1-13

Using the above general method, 12 prochiral diols were converted to the corresponding chiral compounds. In Example 1, the starting compound is not a prochiral compound. The conversion to the monoacetate in each case was excellent. The e.e. in one case was as high as 97:3. The results are shown in Table I.

The monoacetate produced in Example 9 can be used to prepare D-phenylalanine; the monoacetate produced in Example 10 can be used to prepare D-phenylglycine; the monoacetate produced in Example 11 is a useful precursor for (-)-A-factor. The corresponding diols are 2-benzyl-1,3-propanediol, 2-phenyl-1,3-propanediol and 2-(2-propenyl)-1,3-propanediol.

**Table I**

**Conversion of Diols into Monoacetates**

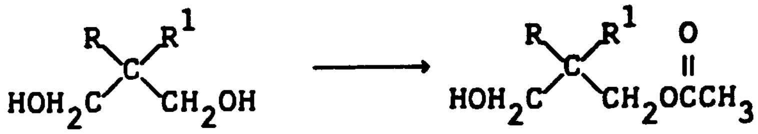

| Example | R | $R^1$ | Percent Conversion | Reaction Time (Hrs.) | e. e. |
|---|---|---|---|---|---|
| 1 | $-CH_3$ | $-CH_3$ | 95 | 48 | – – – |
| 2 | $-CH_3$ | $-H$ | 90.9 | 48 | 55:45 |
| 3 | $-C_2H_5$ | $-H$ | 88 | 24 | 90:10 |
| 4 | $-C_3H_7(n)$ | $-H$ | 90.3 | 48 | 97:3 |
| 5 | $-C_4H_9(n)$ | $-H$ | 89.4 | 42 | 94:6 |
| 6 | $-C_4H_9(t)$ | $-H$ | 63.3 | 17 (days) | 80:20 |
| 7 | 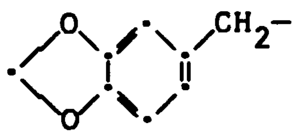 | $-H$ | 76.3 | 168 | 80:20 |
| 8 | $-CH_3$ | $-C_3H_7(n)$ | 84 | 72 | 81:19 |
| 9 | 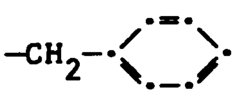 | $-H$ | 88.4 | 64 | 90:10 |
| 10 | 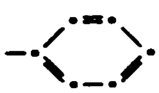 | $-H$ | 81.3 | 96 | 95:5 |
| 11 | $-CH_2CH{=}CH_2$ | $-H$ | 91.7 | 43 | 96:4 |
| 12 | $-CH(CH_3)_2$ | $-H$ | 82.8 | 72 | 96:4 |
| 13 | $-C_2H_5$ | $-C_4H_9(n)$ | 50.6 | 168 | 75:25 |

Examples 14-19

The general procedure described above was used to convert a variety of cis-1,2-bis(hydroxymethyl)-cycloalkanes to the corresponding monoacetates. The results are shown in Table II.

The monoacetate produced in Example 14 is a useful precursor to prepare pyrethrins or chrysanthemic acid. The monoacetate produced in Example 19 is a useful precursor for prostaglandins.

**Table II**

**Conversion of Diols into Monoacetates**

| Example | Diol | Percent Conversion | Reaction Time (Hrs.) | e. e.* |
|---|---|---|---|---|
| 14 | 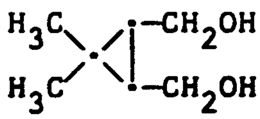 | 12.2 | 90 | 60:40 |
| 15 | 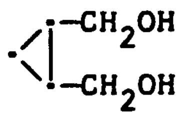 | 75.5 | 90 | 55:45 |
| 16 | 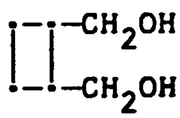 | 74 | 94 | 70:30 |
| 17 | 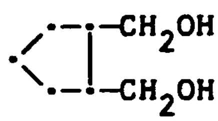 | 27.1 | 90 | 75:25 |
| 18 | 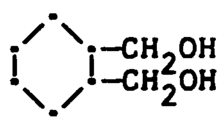 | 16.8 | 90 | 85:15 |
| 19 | 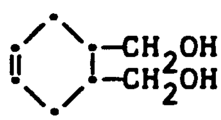 | 23.1 | 90 | 83:17 |

*Emantiomeric excess was calculated at the completion of the reactions, which in some cases may be longer than the listed reaction times.

While the yields were generally lower than with the linear diols, significant amounts of monoacetates were produced using these cyclic diols.

Comparative Example

A number of sources of the biocatalyst were tested. Included in this screen were several different samples of Corynebacterium oxydans and a number of other microorganisms. These other microorganisms were Eschirichia coli (ATCC 11303), Flavobacterium filamentosum (ATCC 31546), Bacillus subtilis (ATCC 31954), Corynebacterium species, Pseudomonas species and Brevibacterium species. Only the Corynebacterium oxydans tested positive in this screen. The microorganisms were C. oxydans ATCC Nos. 21,245; 53586; and 53587.

The reactions were carried out in test tubes containing 5mL of 6% aqueous ethyl acetate, 100 milligrams of 2,2-dimethyl-1,3-propane diol and 100 milligrams of cells. The presence of the monoacetate was determined using thin layer chromatography.

**Claims**

**1.** A method for making a monoacetate from a diol, said method comprising the step of reacting said diol

with an ester of acetic acid in the presence of a biocatalyst derived from Corynebacterium oxydans.

2. A method according to claim 1 wherein said diol is a prochiral compound.

3. A method according to claim 2 wherein said diol is represented by the formula:

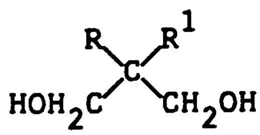

wherein
R and R′ are different and are selected from the group consisting of hydrogen;
substituted or unsubstituted alkyl, alkoxy,
substituted or unsubstituted benzyl,
substituted or unsubstituted aryl and
substituted or unsubstituted heterocyclic.

4. A method according to claim 3 wherein said diol is 2-benzyl-1,3-propanediol, 2-phenyl-1,3-propanediol or 2-(2-propenyl)-1,3-propanediol.

5. A method according to claim 1 wherein said ester of acetic acid is ethyl acetate.

6. A method according to claim 1 wherein the weight ratio of ester of acetic acid to diol is about 5:1 to 100:1.

**Patentansprüche**

1. Verfahren zur Herstellung eines Monoacetates aus einem Diol mit der Stufe der Umsetzung des Diols mit einem Ester der Essigsäure in Gegenwart eines Biokatalysators, der sich von Corynebacterium oxydans ableitet.

2. Verfahren nach Anspruch 1, bei dem das Diol eine prochirale Verbindung ist.

3. Verfahren nach Anspruch 2, bei dem das Diol der folgenden Formel entspricht:

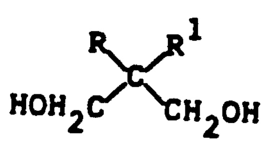

in der
R und $R^1$ verschieden sind und ausgewählt sind aus der Gruppe bestehend aus Wasserstoff; substituiertem oder unsubstituiertem Alkyl, Alkoxy, substituiertem oder unsubstituiertem Benzyl, substituiertem oder unsubstituiertem Aryl und substituierten oder unsubstituierten heterocyclischen Resten.

4. Verfahren nach Anspruch 3, bei dem das Diol 2-Benzyl-1,3-propandiol, 2-Phenyl-1,3-propandiol oder 2-(2-Propenyl)-1,3-propandiol ist.

5. Verfahren nach Anspruch 1, bei dem der Ester der Essigsäure Ethylacetat ist.

6. Verfahren nach Anspruch 1, bei dem das Gewichtsverhältnis des Esters der Essigsäure zum Diol bei etwa 5 : 1 bis 100 : 1 liegt.

**Revendications**

1. Un procédé pour fabriquer un monoacétate à partir d'un diol, ce procédé comprenant l'étape de réaction dudit diol et d'un ester d'acide acétique en présence d'un biocatalyseur dérivé de Corynebacterium oxydans.

2. Un procédé selon la revendication 1, dans lequel ledit diol est un composé prochiral.

3. Un procédé selon la revendication 2, dans lequel ledit diol est représenté par la formule :

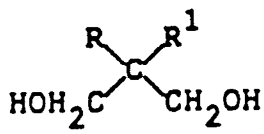

dans laquelle
R et $R^1$ sont différents et sont choisis parmi l'hydrogène, un groupe alkyle substitué ou non, un groupe alcoxy, un groupe benzyle substitué ou non, un groupe aryle substitué ou non et un groupe hétérocyclique substitué ou non.

4. Un procédé selon la revendication 3, dans lequel ledit diol est le 2-benzyl-1,3-propanediol, le 2-phényl-1,3-propanediol ou le 2-(2-propényl)-1,3-propanediol.

5. Un procédé selon la revendication 1, dans lequel ledit ester d'acide acétique est l'acétate d'éthyle.

6. Un procédé selon la revendication 1, dans lequel le rapport pondéral de l'ester d'acide acétique au diol est d'environ 5:1 à 100:1.